# FASCICULE DE BREVET EUROPEEN

(11) **EP 1 181 314 B1**
(45) Date de publication et mention de la délivrance du brevet: **11.11.2009**
(21) Numéro de dépôt: 00936995.0
(22) Date de dépôt: 31.05.2000
(51) Int. Cl.: C07K 14/16, A61K 39/21, C07K 16/10, A61K 39/395

(54) **FRAGMENTS PROTEIQUES POLYEPITOPIQUES DE LA PROTEINE Nef DE HIV, LEUR OBTENTION ET LEURS UTILISATIONS NOTAMMENT EN VACCINATION**
PROTEINFRAGMENTE DIE POLYEPITOPE DES NEF PROTEINS VON HIV UMFASSEN, DEREN HERSTELLUNG UND VERWENDUNGEN INSBESONDERE ZUR IMPFUNG
POLYEPITOPIC PROTEINIC FRAGMENTS OF THE HIV NEF PROTEIN, PRODUCTION AND USE THEREOF IN VACCINATIONS

(30) Priorité: 03.06.1999 FR 9907012
(43) Date de publication de la demande: 27.02.2002
(73) Titulaire: Inserm-Transfert SA, 75013 Paris (FR)
(72) Inventeur: CHOPPIN, Jeannine, F-93110 Rosny-Sous-Bois (FR); BOURGAULT VILLADA, Isabelle, F-75007 Paris (FR); GUILLET, Jean-Gérard, F-75009 Paris (FR); CONNAN, Francine, F-95170 Deuil la Barre (FR); FERRIES, Estelle, F-75013 Paris (FR)
(74) Mandataire: Grosset-Fournier, Chantal Catherine
(86) Numéro de dépôt international: PCT/FR2000/001514
(87) Numéro de publication internationale: WO 2000/075181

(56) Documents cités:
- EP-A- 0 728 764
- TORRES B A ET AL: "IDENTIFICATION OF AN HIV-1 NEF PEPTIDE THAT BINDS TO HLA CLASS II ANTIGENS" BIOCHEMICAL AND BIOPHYSICAL RESEARCH COMMUNICATIONS,US,ACADEMIC PRESS INC. ORLANDO, FL, vol. 200, no. 2, 29 avril 1994 (1994-04-29), pages 1059-1065, XP000578181 ISSN: 0006-291X
- CULMANN B ET AL: "Identification of multirestricted immunodominant regions recognized by cytolytic T lymphocytes in the Human Immunodeficiency Virus type 1 Nef protein" JOURNAL OF VIROLOGY, vol. 68, no. 11, novembre 1994 (1994-11), pages 7336-7343, XP002132359 AMERICAN SOCIETY FOR MICROBIOLOGY US
- HUNZIKER I P ET AL: "Who is right? Or, how to judge the disagreement about HLA restriction of Nef peptides" AIDS RESEARCH AND HUMAN RETROVIRUSES, vol. 14, no. 11, 20 juillet 1998 (1998-07-20), pages 921-923, XP002132360 ISSN:0889-2229

### LISTE DE SEQUENCES

<110> BIOVECTOR THERAPEUTICS INSERM
<120> FRAGMENTS PROTEIQUES POLYEPITOPIQUES DE LA PROTEINE NEF, LEUR OBTENTION ET LEURS UTILISATIONS
<130> WOB EPIT NEF
<140>
   <141>
<150> FR9907012
   <151> 1999-06-03
<160> 8
<170> PatentIn Ver. 2.1
<210> 1
   <211> 618
   <212> ADN
   <213> Human Immuno Deficiency Virus
<220>
   <221> CDS
   <222> (1)..(618)
<400> 1
   atg ggt ggc aag tgg tca aaa agt agt gtg gtt gga tgg cct act gta      48
   Met Gly Gly Lys Trp Ser Lys Ser Ser Val Val Gly Trp Pro Thr Val
    1               5                  10                  15
   agg gaa aga atg aga cga gct gag cca gca gca gat ggg gtg gga gca      96
   Arg Glu Arg Met Arg Arg Ala Glu Pro Ala Ala Asp Gly Val Gly Ala
               20                  25                  30
   gca tct cga gac ctg gaa aaa cat gga gca atc aca agt agc aat aca     144
   Ala Ser Arg Asp Leu Glu Lys His Gly Ala Ile Thr Ser Ser Asn Thr
           35                  40                  45
   gca gct acc aat gct gct tgt gcc tgg cta gaa gca caa gag gag gag     192
   Ala Ala Thr Asn Ala Ala Cys Ala Trp Leu Glu Ala Gln Glu Glu Glu
       50                  55                  60
   gag gtg ggt ttt cca gtc aca cct cag gta cct tta aga cca atg act     240
   Glu Val Gly Phe Pro Val Thr Pro Gln Val Pro Leu Arg Pro Met Thr
   65                  70                  75                  80
   tac aag gca gct gta gat ctt agc cac ttt tta aaa gaa aag ggg gga     288
   Tyr Lys Ala Ala Val Asp Leu Ser His Phe Leu Lys Glu Lys Gly Gly
                   85                  90                  95
   ctg gaa ggg cta att cac tcc caa cga aga caa gat atc ctt gat ctg     336
   Leu Glu Gly Leu Ile His Ser Gln Arg Arg Gln Asp Ile Leu Asp Leu
               100                 105                 110
   tgg atc tac cac aca caa ggc tac ttc cct gat tgg cag aac tac aca     384
   Trp Ile Tyr His Thr Gln Gly Tyr Phe Pro Asp Trp Gln Asn Tyr Thr
           115                 120                 125
   cca ggg cca ggg gtc aga tat cca ctg acc ttt gga tgg tgc tac aag      432
   Pro Gly Pro Gly Val Arg Tyr Pro Leu Thr Phe Gly Trp Cys Tyr Lys
       130                 135                 140
   cta gta cca gtt gag cca gat aag gta gaa gag gcc aat aaa gga gag      480
   Leu Val Pro Val Glu Pro Asp Lys Val Glu Glu Ala Asn Lys Gly Glu
   145                 150                 155                 160
   aac acc agc ttg tta cac cct gtg agc ctg cat gga atg gat gac cct      528
   Asn Thr Ser Leu Leu His Pro Val Ser Leu His Gly Met Asp Asp Pro
                   165                 170                 175
   gag aga gaa gtg tta gag tgg agg ttt gac agc cgc cta gca ttt cat      576
   Glu Arg Glu Val Leu Glu Trp Arg Phe Asp Ser Arg Leu Ala Phe His
               180                 185                 190
   cac gtg gcc cga gag ctg cat ccg gag tac ttc aag aac tgc              618
   His Val Ala Arg Glu Leu His Pro Glu Tyr Phe Lys Asn Cys
           195                 200                 205
<210> 2
   <211> 206
   <212> PRT
   <213> Human Immuno Deficiency Virus
<400> 2
   Met Gly Gly Lys Trp Ser Lys Ser Ser Val Val Gly Trp Pro Thr Val
   1 5 10 15
   Arg Glu Arg Met Arg Arg Ala Glu Pro Ala Ala Asp Gly Val Gly Ala
   20 25 30
   Ala Ser Arg Asp Leu Glu Lys His Gly Ala Ile Thr Ser Ser Asn Thr
   35 40 45
   Ala Ala Thr Asn Ala Ala Cys Ala Trp Leu Glu Ala Gln Glu Glu Glu
   50 55 60
   Glu Val Gly Phe Pro Val Thr Pro Gln Val Pro Leu Arg Pro Met Thr
   65 70 75 80
   Tyr Lys Ala Ala Val Asp Leu Ser His Phe Leu Lys Glu Lys Gly Gly
   85 90 95
   Leu Glu Gly Leu Ile His Ser Gln Arg Arg Gln Asp Ile Leu Asp Leu
   100 105 110
   Trp Ile Tyr His Thr Gln Gly Tyr Phe Pro Asp Trp Gln Asn Tyr Thr
   115 120 125
   Pro Gly Pro Gly Val Arg Tyr Pro Leu Thr Phe Gly Trp Cys Tyr Lys
   130 135 140
   Leu Val Pro Val Glu Pro Asp Lys Val Glu Glu Ala Asn Lys Gly Glu
   145 150 155 160
   Asn Thr Ser Leu Leu His Pro Val Ser Leu His Gly Met Asp Asp Pro
   165 170 175
   Glu Arg Glu Val Leu Glu Trp Arg Phe Asp Ser Arg Leu Ala Phe His
   180 185 190
   His Val Ala Arg Glu Leu His Pro Glu Tyr Phe Lys Asn Cys
   195 200 205
<210> 3
   <211> 90
   <212> ADN
   <213> Séquence artificielle
<220>
   <223> Description de la séquence artificielle: fragment de la séquence codant pour Nef du virus HIV et séquence peptidique correspondante
<220>
   <221> CDS
   <222> (1)..(90)
<400> 3
   ttt cca gtc aca cct cag gta cct tta aga cca atg act tac aag gca    48
   Phe Pro Val Thr Pro Gln Val Pro Leu Arg Pro Met Thr Tyr Lys Ala
     1               5                  10                  15
   gct gta gat ctt agc cac ttt tta aaa gaa aag ggg gga ctg            90
   Ala Val Asp Leu Ser His Phe Leu Lys Glu Lys Gly Gly Leu
               20                  25                  30
<210> 4
   <211> 30
   <212> PRT
   <213> Séquence artificielle
   <223> Description de la séquence artificielle: fragment de la séquence codant pour Nef du virus HIV et séquence peptidique correspondante
<400> 4
   Phe Pro Val Thr Pro Gln Val Pro Leu Arg Pro Met Thr Tyr Lys Ala
     1               5                  10                  15
   Ala Val Asp Leu Ser His Phe Leu Lys Glu Lys Gly Gly Leu
               20                  25                  30
<210> 5
   <211> 90
   <212> ADN
   <213> Séquence artificielle
<220>
   <223> Description de la séquence artificielle: fragment de la séquence codant pour Nef de HIV, et séquence peptidique correspondante
<220>
   <221> CDS
   <222> (1)..(90)
<400> 5
   cac aca caa ggc tac ttc cct gat tgg cag aac tac aca cca ggg cca    48
   His Thr Gln Gly Tyr Phe Pro Asp Trp Gln Asn Tyr Thr Pro Gly Pro
     1               5                  10                  15
   ggg gtc aga tat cca ctg acc ttt gga tgg tgc tac aag cta            90
   Gly Val Arg Tyr Pro Leu Thr Phe Gly Trp Cys Tyr Lys Leu
               20                  25                  30
<210> 6
   <211> 30
   <212> PRT
   <213> Séquence artificielle
   <223> Description de la séquence artificielle: fragment de la séquence codant pour Nef de HIV, et séquence peptidique correspondante
<400> 6
   His Thr Gln Gly Tyr Phe Pro Asp Trp Gln Asn Tyr Thr Pro Gly Pro
   1 5 10 15
   Gly Val Arg Tyr Pro Leu Thr Phe Gly Trp Cys Tyr Lys Leu
   20 25 30
<210> 7
   <211> 72
   <212> ADN
   <213> Séquence artificielle
<220>
   <223> Description de la séquence artificielle: fragment de la séquence codant pour Nef de HIV et séquence peptidique correspondante
<220>
   <221> CDS
   <222> (1)..(72)
<400> 7
   gtg tta gag tgg agg ttt gac agc cgc cta gca ttt cat cac gtg gcc 48
   Val Leu Glu Trp Arg Phe Asp Ser Arg Leu Ala Phe His His Val Ala
   1 5 10 15
   cga gag ctg cat ccg gag tac ttc 72
   Arg Glu Leu His Pro Glu Tyr Phe
   20
<210> 8
   <211> 24
   <212> PRT
   <213> Séquence artificielle
   <223> Description de la séquence artificielle: fragment de la séquence codant pour Nef de HIV et séquence peptidique correspondante
<400> 8
   Val Leu Glu Trp Arg Phe Asp Ser Arg Leu Ala Phe His His Val Ala
   1 5 10 15
   Arg Glu Leu His Pro Glu Tyr Phe
   20

## Description

La présente invention a pour objet des fragments protéiques polyépitopiques, tels que ceux de la protéine Nef du virus HIV du SIDA, leur procédé d'obtention, et leurs utilisations, notamment dans le domaine de la vaccination thérapeutique ou préventive.

L'invention a plus particulièrement pour objet l'utilisation de fragments polyépitopiques d'une protéine déterminée pour la préparation de médicaments destinés à la prévention ou au traitement de pathologies dans lesquelles ladite protéine est reconnue par le système immunitaire cellulaire.

Avantageusement, lesdits fragments polyépitopiques sont tels que leur acide aminé N-terminal correspond à l'acide aminé N-terminal de l'épitope situé en amont d'un ou plusieurs autres épitopes d'une région polyépitopique de ladite protéine, et leur acide aminé C-terminal correspond à l'acide aminé C-terminal de l'épitope situé en aval du ou des épitopes susmentionnés de ladite région polyépitopique.

Ainsi, les fragments protéiques polyépitopiques susmentionnés de la présente invention correspondent avantageusement aux régions polyépitopiques d'une protéine déterminée, à savoir aux régions contenant plusieurs épitopes reconnus par les cellules T en association avec les différentes molécules du complexe majeur d'histocompatibilité (CMH), lesdites régions étant sélectionnées parmi celles ayant la caractéristique d'être dégradées *in vitro* en peptides plus courts par des protéasomes, tel que le protéasome 20S, lorsque le fragment protéique testé est mis en présence dudit protéasome, notamment selon la méthode détaillée suivante. Le fragment protéique (environ 75 µg lorsqu'il s'agit d'un polypeptide d'environ 30 acides aminés) est incubé à 37°C avec environ 15µg de protéasome 20S (Calbiochem Ref 539150, La Jolla, CA, USA) dans 500 µl du tampon suivant : 20 mM Tris-HCl pH8, 0,5 mM EDTA. Des aliquots de 50 µl sont prélevés après des temps d'incubation de 24 et 48 heures, et sont analysés par chromatographie liquide haute pression (HPLC). Les produits de digestion des protéasomes sont séparés par RP-HPLC (Perkin Elmer) en utilisant une colonne C18 et un gradient acétonitrile (de 0 à 100 % contenant 0,1 % d'acide trifluoroacétique, en 90 mn, taux d'élution 0,8 ml/mn). Les produits de clivage sont détectés à 214 nm par un détecteur à absorption (759A, Applied Biosystems).

Avantageusement les régions polyépitopiques définies ci-dessus possèdent la caractéristique de contenir des acides aminés hydrophobes.

Les différents épitopes de la région polyépitopique de la protéine déterminée, et délimitant les fragments protéiques polyépitopiques, sont avantageusement sélectionnés parmi les peptides ;
- se liant à une molécule déterminée du CMH, notamment à une molécule de type HLA déterminé, et ce jusqu'à des concentrations d'environ 10⁻⁶ M à environ 10⁻¹⁰ M en peptide pour des concentrations d'environ 10⁻⁷ M en molécule HLA, notamment dans les conditions décrites ci-après,
- et formant un complexe stable avec cette molécule du CMH, à savoir notamment un complexe dans lequel ledit peptide reste lié à ladite molécule pendant au moins environ 3 heures à 37°C.

A titre d'illustration, les épitopes susmentionnés de l'invention sont sélectionnés parmi les peptides susceptibles :
- d'une part de s'associer avec les molécules du CMH, notamment par mise en oeuvre de la méthode suivante :
   - incubation (notamment pendant environ 2 heures à 25°C, puis environ 15 heures à 4°C) du peptide en présence de molécules du CMH, provenant de la lyse de cellules humaines ou animales, ou purifiées notamment par chromatographie d'affinité à partir de lignées cellulaires humaines ou animales,
   - piégeage des complexes formés lors de l'étape précédente sur un support solide recouvert d'un premier anticorps, notamment monoclonal, reconnaissant spécifiquement les molécules du CMH dans leur conformation dépendante de leur liaison audit peptide,
   - addition sur le support solide précédent d'un deuxième anticorps marqué, notamment par couplage à un marqueur radioactif, enzymatique ou fluorescent, ledit anticorps marqué reconnaissant spécifiquement soit les chaînes lourdes du CMH dans leur conformation dépendante de leur liaison au peptide, soit la chaîne légère du CMH
      ou la β2-microglobuline se liant spécifiquement aux différentes chaînes lourdes du CMH dans leur conformation susmentionnée,
   - détection, après rinçage du support solide, de l'éventuelle présence du deuxième anticorps marqué resté fixé sur le support solide, témoignant d'un effet d'association entre les molécules du CMH et le peptide étudié,
- et, d'autre part, de former un complexe avec lesdites molécules du CMH, dont la stabilité peut être évaluée par mise en oeuvre d'une méthode de suivi dans le temps de la liaison établie entre le peptide et les molécules du CMH, cette méthode étant avantageusement effectuée selon un protocole identique à la méthode précédente, mais dans laquelle l'étape d'incubation du peptide en présence des molécules du CMH sur le support solide recouvert dudit premier anticorps, est précédée par une étape préalable d'élimination du peptide libre susceptible d'être présent dans le milieu réactionnel, notamment par lavage du support solide, ladite étape d'incubation étant effectuée (avantageusement à une température de 37°C) pendant des temps variables de 1h, 3h, 5h, 24h et 48h.

Le document Culman et al. ("Identification of multirestricted immunodominant regions recognized by cytolytic T lymphocytes in the human immunodeficiency virus types 1 Nef protein" Journal of Virology, Vol. 68, n° 11, novembre 1994, pages 7336-7343, American Society for Microbiology US) décrit l'identification de régions immunoprédominantes de la protéine Nef du VIH de type 1 reconnues par des lymphocytes T cytotoxiques.

Comme mentionné ci-dessus, les épitopes de l'invention doivent être reconnus par les cellules T en association avec les molécules du CMH et s'associer à ces dernières, notamment dans le cadre de la mise en oeuvre du test de reconnaissance décrit ci-dessus. Cette association peut être faible (détectable à des concentrations en analogues peptidiques de l'ordre de 10⁻⁴ à 10⁻⁵ M), intermédiaire (détectable à des concentrations en analogues peptidiques de l'ordre de 10⁻⁶ à 10⁻⁷ M), ou forte (détectable à des concentrations en analogues peptidiques de l'ordre de 10⁻⁸ à 10⁻⁹ M).

Les peptides associés aux molécules du CMH dans le cadre de la présente invention sont de préférence susceptibles de se lier pendant au moins environ 3 heures auxdites molécules du CMH.

L'invention a plus particulièrement pour objet les épitopes (encore désignés peptides ci-dessus et ci-après) tels que décrits ci-dessus et **caractérisés en ce qu**'ils sont sélectionnés parmi ceux susceptibles :
- d'induire *in vitro* la cytolyse par des lymphocytes T cytotoxiques, de cellules cibles présentant à leur surface le peptide susmentionné associé aux molécules du CMH, lesdits lymphocytes T cytotoxiques étant avantageusement prélevés sur un patient atteint d'une pathologie dans laquelle est impliqué le peptide étudié,
- et d'induire *in vitro* la sécrétion de cytokines (ou interleukines) par les lymphocytes T cytotoxiques susmentionnés, notamment IL-2, IL-4 ou l'interféron γ.

Le cas échéant, les épitopes susmentionnés sont choisis parmi ceux capables d'induire *in vitro* l'apparition et la croissance de lymphocytes T cytotoxiques à partir de cellules humaines ou animales, notamment à partir de cellules mononucléées issues du sang périphérique (PBMC), en présence de facteurs nécessaires à la croissance et la différenciation des cellules T cytotoxiques.

Les fragments protéiques polyépitopiques de l'invention sont davantage **caractérisés en ce qu**'ils sont susceptibles de contenir des épitopes CD4 reconnus par les cellules T auxiliaires en association avec les molécules du CMH de classe II, cette propriété favorisant l'induction et le maintien des cellules T CD8⁺ reconnaissant les épitopes compris dans lesdits fragments.

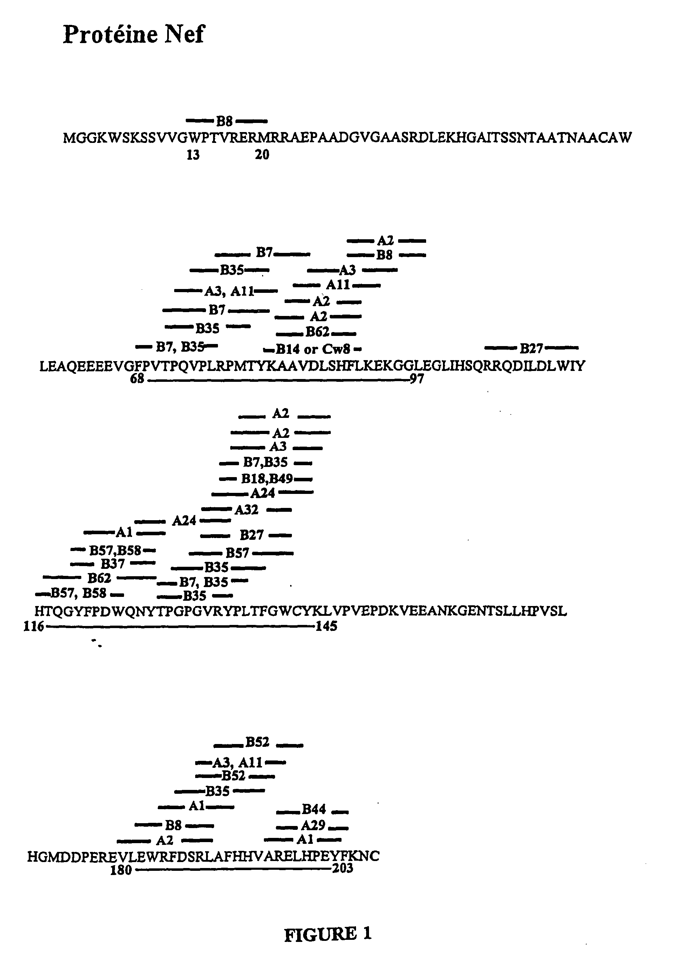

La présente invention est illustrée à l'aide de la figure 1 représentant la séquence peptidique de la protéine Nef de HIV, ainsi que les fragments polyépitopiques de l'invention, et les épitopes au sein de ces fragments.

L'invention a plus particulièrement pour objet le fragment polyépitopique de la protéine Nef de HIV tel que défini ci-dessus, **caractérisé en ce qu**'il correspond au fragment de 30 acides aminés délimité par les acides aminés situés aux positions 68 et 97 de la séquence peptidique de la protéine Nef, et caractérisé par la séquence peptidique SEQ ID NO : 4 suivante:
(68)FPVTPQVPLRPMTYKAAVDLSHFLKEKGGL(97)
ledit fragment contenant 13 épitopes se liant de façon stable à l'une au moins des 9 molécules HLA de types suivants : A2, A3, A11, B7, B8, B14, Cw8, B35, et B62, lesdits épitopes étant les suivants :
- (68)FPVTPQVPL(76) se liant de façon stable aux molécules HLA de type B7 ou B35,
- (71)TPQVPLRPM(79) se liant de façon stable aux molécules HLA de type B35, ou B7,
- (71)TPQVPLRPMTY(81) se liant de façon stable aux molécules HLA de type B7, ou B35,
- (73)QVPLRPMTYK(82) se liant de façon stable aux molécules HLA de type A3 ou A11,
- (74)VPLRPMTY(81) se liant de façon stable aux molécules HLA de type B35,
- (77)RPMTYKAAV(85) se liant de façon stable aux molécules HLA de type B7,
- (82)KAAVDLSHFL(91) se liant de façon stable aux molécules HLA de type B14 ou Cw8,
- (83)AAVDLSHF(90) se liant de façon stable aux molécules HLA de type B62,
- (83)AAVDLSHFL(91) se liant de façon stable aux molécules HLA de type A2,
- (84)AVDLSHFL(91) se liant de façon stable aux molécules HLA de type A2,
- (84)AVDLSHFLK(92) se liant de façon stable aux molécules HLA de type A11,
- (86)DLSHFLKEK(94) se liant de façon stable aux molécules HLA de type A3,
- (90)FLKEKGGL(97) se liant de façon stable aux molécules HLA de type A2 ou B8.

L'invention a également pour objet le fragment polyépitopique de la protéine Nef de HIV tel que défini ci-dessus, **caractérisé en ce qu**'il correspond au fragment de 30 acides aminés délimité par les acides aminés situés aux positions 116 et 145 de la séquence peptidique de la protéine Nef, et caractérisé par la séquence peptidique SEQ ID NO : 6 suivante:
(116)HTQGYFPDWQNYTPGPGVRYPLTFGWCYKL(145)
ledit fragment contenant 15 épitopes se liant de façon stable à l'une au moins des 14 molécules HLA de types suivants : A1, A2, A3, A24, A32, B7, B18, B27, B35, B37, B49, B57, B58, ou B62, lesdits épitopes étant les suivants :
- (116)HTQGYFPDW(124) se liant de façon stable aux molécules HLA de type B57 ou B58,
- (117)TQGYFPDWQNY(127) se liant de façon stable aux molécules HLA de type B62,
- (120)YFPDWQNY(127) se liant de façon stable aux molécules HLA de type B37, B57 ou B58,
- (121)FPDWQNYT(128) se liant de façon stable aux molécules HLA de type A1,
- (126)NYTPGPGVRY(135) se liant de façon stable aux molécules HLA de type A24,
- (128)TPGPGVRY(135) se liant de façon stable aux molécules HLA de type B35,
- (128)TPGPGVRYPL(137) se liant de façon stable aux molécules HLA de type B7 ou B35,
- (130)GPGVRYPLTF(139) se liant de façon stable aux molécules HLA de type B35, ou B7,
- (132)GVRYPLTFGW(141) se liant de façon stable aux molécules HLA de type B57,
- (133)VRYPLTFGW(141) se liant de façon stable aux molécules HLA de type A32 ou B27,
- (134)RYPLTFGWCY(143) se liant de façon stable aux molécules HLA de type A24,
- (135)YPLTFGWCY(143) se liant de façon stable aux molécules HLA de type B7, B18, B35, ou B49,
- (136)PLTFGWCYK(144) se liant de façon stable aux molécules HLA de type A3,
- (136)PLTFGWCYKL(145) se liant de façon stable aux molécules HLA de type A2,
- (137)LTFGWCYKL(145) se liant de façon stable aux molécules HLA de type A2.

L'invention concerne également le fragment polyépitopique de la protéine Nef de HIV tel que défini ci-dessus, **caractérisé en ce qu**'il correspond au fragment de 24 acides aminés délimité par les acides aminés situés aux positions 180 et 203 de la séquence peptidique de la protéine Nef, et caractérisé par la séquence peptidique SEQ ID NO : 8 suivante:
(180)VLEWRFDSRLAFHHVARELHPEYF(203)
ledit fragment contenant 9 épitopes se liant de façon stable à l'une au moins des 9 molécules HLA de types suivants : A1, A2, A3, A11, A29, B8, B35, B44, ou B52, lesdits épitopes étant les suivants :
- (180)VLEWRFDSRL(189) se liant de façon stable aux molécules HLA de type A2,
- (182)EWRFDSRL(189) se liant de façon stable aux molécules HLA de type B8,
- (184)RFDSRLAF(191) se liant de façon stable aux molécules HLA de type A1,
- (186)DSRLAFHHV(194) se liant de façon stable aux molécules HLA de type B35,
- (188)RLAFHHVA(195) se liant de façon stable aux molécules HLA de type B52,
- (188)RLAFHHVAR(196) se liant de façon stable aux molécules HLA de type A3 ou A11,
- (190)AFHHVAREL(198) se liant de façon stable aux molécules HLA de type B52,
- (195)ARELHPEY(202) se liant de façon stable aux molécules HLA de type A1,
- (196)RELHPEYF(203) se liant de façon stable aux molécules HLA de type A29 ou B44.

L'invention a également pour objet les séquences nucléotidiques, choisies parmi les suivantes:
- la séquence SEQ ID NO : 3, codant pour le fragment polyépitopique SEQ ID NO : 4 susmentionné,
- la séquence SEQ ID NO : 5, codant pour le fragment polyépitopique SEQ ID NO : 6 susmentionné,
- la séquence SEQ ID NO : 7, codant pour le fragment polyépitopique SEQ ID NO : 8 susmentionné.

L'invention a également pour objet tout vecteur, notamment plasmide, cosmide ou phage, contenant au moins une séquence nucléotidique susmentionnée placée sous le contrôle des éléments nécessaires à la transcription de ladite séquence, notamment sous le contrôle d'un promoteur et d'un terminateur de transcription.

L'invention concerne également les cellules hôtes, notamment bactéries, virus, levures, cellules eucaryotes, transformées à l'aide d'un vecteur susmentionné selon l'invention, de manière à intégrer de façon stable dans leur génome ou à maintenir de manière stable dans leur cytoplasme, au moins une séquence nucléotidique selon l'invention.

L'invention concerne également tout vecteur comprenant un ou plusieurs fragments tels que définis ci-dessus, ou tout vecteur comprenant une ou plusieurs séquences nucléotidiques susmentionnées, lesdits vecteurs étant choisis parmi ceux capables d'assurer une protection desdits fragments ou séquences nucléotidiques dans l'organisme et/ou leur pénétration dans les cellules de l'organisme.

Dans le cas de l'utilisation de fragments polyépitopiques et/ou de séquences peptidiques dérivées susmentionnés, de tels vecteurs sont choisis parmi les acides gras (dans le cadre de la préparation de lipopeptides), les liposomes etc.

A ce titre, l'invention a plus particulièrement pour objet tout lipopeptide **caractérisé en ce qu**'il comprend:
- une partie peptidique comprenant un ou plusieurs fragments ou sous-fragments protéiques polyépitopiques choisis parmi ceux définis ci-dessus, ou toute séquence peptidique dérivée desdits fragments telle que définie ci-dessus,
- et une ou plusieurs parties lipophiles, avantageusement choisies parmi celles comprenant :
   * une chaîne hydrocarbonée en C4 à C20, saturée ou insaturée, linéaire ou ramifiée,
   * ou un groupe stéroïde, le cas échéant lié à la chaîne hydrocarbonée susmentionnée,
lesdites parties lipophiles étant éventuellement associées à un court peptide vecteur (pour former ainsi des motifs lipopeptidiques vecteurs) comportant une ou plusieurs fonctions ionisées à pH physiologique, et une fonction permettant la fixation covalente de ladite chaîne hydrocarbonée et/ou dudit groupe stéroïde.

Par partie lipophile, dans ce qui précède et ce qui suit, on entend toute molécule lipophile, insoluble dans l'eau, permettant, lorsqu'elle est liée à la partie peptidique définie ci-dessus, un passage intracellulaire passif du lipopeptide obtenu, grâce aux propriétés hydrophobes de ladite molécule. Avantageusement le lipopeptide résultant de la liaison de la partie lipophile à la partie peptidique, est soluble dans l'eau.

De préférence, la chaîne hydrocarbonée des parties lipophiles, est choisie parmi celles de :
- l'acide palmitique,
- l'acide oléique,
- l'acide linoléique,
- l'acide linolénique.

De préférence également, le groupe stéroïde de la ou des parties lipophiles, est choisi parmi les dérivés du cholestérol tel que l'acide cholest-5-ényl-3-oxy acétique, ou l'acide cholest-5-ényl-3-oxycarbonique.

L'invention a plus particulièrement pour objet tout lipopeptide tel que décrit ci-dessus, **caractérisé en ce que** la ou les parties lipophiles sont liées de façon covalente à un ou plusieurs acides aminés de la partie peptidique.

Avantageusement, la ou les parties lipophiles sont liées de façon covalente à la fonction αNH₂ ou εNH₂ d'une lysine située en position N-terminale ou C-terminale de la partie peptidique, ou à la fonction thiol d'une cystéine, ou à toute fonction amino, alcool ou thiol éventuellement ajoutée au peptide avec un espaceur simple.

A ce titre, l'invention a plus particulièrement pour objet tout lipopeptide tel que défini ci-dessus, dans lequel la ou les parties lipophiles sont représentées par un groupe N^{α}-acétyl-Lysine N^{ε}(palmitoyl) (encore désigné par l'abréviation Ac-K(Pam)).

La présente invention a également pour objet, des micelles ou micro-agrégats d'un
ou plusieurs lipopeptides différents définis ci-dessus.

Avantageusement, lesdits micelles ou micro-agrégats ont une taille inférieure à environ 1µm.

De préférence, les micelles ou micro-agrégats selon l'invention, sont tels qu'obtenus par dispersion desdits lipopeptides dans une solution d'acide acétique concentrée à environ 80%, ou tout autre solvant capable d'assurer une dispersion moléculaire des lipopeptides en solution.

Dans le cas de l'utilisation de séquences nucléotidiques définies ci-dessus selon l'invention, les vecteurs susmentionnés sont choisis parmi les virus, notamment les rétrovirus, les adénovirus et les virus associés (AAV Adeno Associated Virus).

L'invention a également pour objet l'utilisation :
- d'au moins un fragment polyépitopique de la protéine Nef de HIV, choisis parmi ceux définis ci-dessus, le cas échéant en association avec un ou plusieurs fragments polyépitopiques d'autres protéines, que la protéine Nef, du virus HIV, notamment d'un ou plusieurs fragments polyépitopiques de la protéine GAG et/ou POL et/ou ENV et/ou RT,
- et/ou d'au moins une séquence peptidique dérivée de ce fragment, telle que définie ci-dessus, le cas échéant en association avec un ou plusieurs fragments polyépitopiques d'autres protéines susmentionnées,
- ou d'au moins une séquence nucléotidique, telle que définie ci-dessus, codant pour un fragment ou sous-fragment polyépitopique de la protéine Nef de HIV, et/ou pour une séquence peptidique dérivée de ce fragment, tels que définis ci-dessus,
pour la préparation d'un médicament ou vaccin destiné à la prévention ou au traitement du SIDA.

A ce titre, l'invention concerne également les compositions pharmaceutiques, ou les vaccins, **caractérisés en ce qu**'ils comprennent :
- au moins un fragment polyépitopique de la protéine Nef, tels que définis ci-dessus,
- et/ou des lipopeptides et/ou micelles définis ci-dessus, contenant au moins un fragment ou sous-fragment polyépitopique susmentionné de la protéine Nef,
   en association avec un véhicule physiologiquement acceptable,
   ledit fragment ou sous-fragment protéique polyépitopique et/ou sa séquence dérivée étant le cas échéant associés à un ou plusieurs autres épitopes exogènes reconnus par des cellules T auxiliaires (encore désignés épitopes CD4 ou T helper), lesdits épitopes étant choisis notamment parmi les suivants :
- le fragment peptidique délimité par les acides aminés situés aux positions 830 et 846 de la séquence peptidique de la toxine tétanique, ledit fragment répondant à la formule suivante : QYIKANSKFIGITELKK,
- l'hémagglutinine (Prevost-Blondel et al., 1995, J. Virol., 62, n°12, pp 8046-8055),
- l'épitope PADRE (Alexander et al., 1994, Immunity, 1, 751).

L'invention concerne également les compositions pharmaceutiques, ou les vaccins, **caractérisés en ce qu**'ils comprennent :
- au moins une séquence nucléotidique codant pour un fragment polyépitopique de la protéine Nef, telle que définie ci-dessus,
- et/ou au moins un vecteur approprié susmentionné, choisi notamment parmi les virus définis ci-dessus, contenant au moins une séquence nucléotidique susmentionnée,
en association avec un véhicule physiologiquement acceptable.

Avantageusement, les compositions pharmaceutiques, ou vaccins, susmentionnés, se présentent sous une forme administrable par voie sous-cutanée ou intramusculaire, notamment à raison de plusieurs injections (avantageusement 3 injections) d'environ 500 µg du fragment polyépitopique sous forme lipopeptidique, à environ un mois d'intervalle.

Les fragments polyépitopiques, les épitopes simples (peptides susmentionnés), ou les séquences dérivées, de la présente invention peuvent être obtenus par synthèse peptidique classique en phase liquide ou en phase solide.

## Revendications

1. Fragment polyépitopique de la protéine Nef de HIV **caractérisés en ce qu'**il correspondent :
* au fragment de 30 acides aminés délimité par les acides aminés situés aux positions 68 et 97 de la séquence peptidique de la protéine Nef, et **caractérisé par** la séquence peptidique SEQ ID NO : 4 suivante:
(68)FPVTPQVPLRPMTYKAAVDLSHFLKEKGGL(97)
ledit fragment contenant 13 épitopes se liant de façon stable à l'une au moins des 9 molécules HLA de types suivants : A2, A3, A11, B7, B8, B14, Cw8, B35, et B62, lesdits épitopes étant les suivants :
- (68)FPVTPQVPL(76) se liant de façon stable aux molécules HLA, de type B7 ou B35,
- (71)TPQVPLRPM(79) se liant de façon stable aux molécules HLA de type B35, ou B7,
- (71)TPQVPLRPMTY(81) se liant de façon stable aux molécules HLA de type B7, ou B35,
- (73)QVPLRPMTYK(82) se liant de façon stable aux molécules HLA de type A3 ou A11,
- (74)VPLRPMTY(81) se liant de façon stable aux molécules HLA de type B35,
- (77)RPMTYKAAV(85) se liant de façon stable aux molécules HLA de type B7,
- (82)KAAVDLSHFL(91) se liant de façon stable aux molécules HLA de type B14 ou Cw8,
- (83)AAVDLSHF(90) se liant de façon stable aux molécules HLA de type B62,
- (83)AAVDLSHFL(91) se liant de façon stable aux molécules HLA de type A2,
- (84)AVDLSHFL(91) se liant de façon stable aux molécules HLA de type A2,
- (84)AVDLSHFLK(92) se liant de façon stable aux molécules HLA de type A11,
- (86)DLSHFLKEK(94) se liant de façon stable aux molécules HLA de type A3,
- (90)FLKEKGGL(97) se liant de façon stable aux molécules HLA de type A2 ou B8.
* au fragment de 30 acides aminés délimité par les acides aminés situés aux positions 116 et 145 de la séquence peptidique de la protéine Nef, et **caractérisé par** la séquence peptidique SEQ ID NO : 6 suivante:
(116)HTQGYFPDWQNYTPGPGVRYPLTFGWCYKL(145)
ledit fragment contenant 15 épitopes se liant de façon stable à l'une au moins des 14 molécules HLA de types suivants : A1, A2, A3, A24, A32, B7, B18, B27, B35, B37, B49, B57, B58, ou B62, lesdits épitopes étant les suivants :
- (116)HTQGYFPDW(124) se liant de façon stable aux molécules HLA de type B57 ou B58,
- (117)TQGYFPDWQNY(127) se liant de façon stable aux molécules HLA de type B62,
- (120)YFPDWQNY(127) se liant de façon stable aux molécules HLA de type B37, B57 ou B58,
- (121)FPDWQNYT(128) se liant de façon stable aux molécules HLA de type A1,
- (126)NYTPGPGVRY(135) se liant de façon stable aux molécules HLA de type A24,
- (128)TPGPGVRY(135) se liant de façon stable aux molécules HLA de type B35,
- (128)TPGPGVRYPL(137) se liant de façon stable aux molécules HLA de type B7 ou B35,
- (130)GPGVRYPLTF(139) se liant de façon stable aux molécules HLA de type B35, ou B7,
- (132)GVRYPLTFGW(141) se liant de façon stable aux molécules HLA de type B57,
- (133)VRYPLTFGW(141) se liant de façon stable aux molécules HLA de type A32 ou B27,
- (134)RYPLTFGWCY(143) se liant de façon stable aux molécules HLA de type A24,
- (135)YPLTFGWCY(143) se liant de façon stable aux molécules HLA de type B7, B18, B35, ou B49,
- (136)PLTFGWCYK(144) se liant de façon stable aux molécules HLA de type A3,
- (136)PLTFGWCYKL(145) se liant de façon stable aux molécules HLA de type A2,
- (137)LTFGWCYKL(145) se liant de façon stable aux molécules HLA de type A2.
* au fragment de 24 acides aminés délimité par les acides aminés situés aux positions 180 et 203 de la séquence peptidique de la protéine Nef, et **caractérisé par** la séquence peptidique SEQ ID NO : 8 suivante:
(180)VLEWRFDSRLAFHHVARELHPEYF(203)
ledit fragment contenant 9 épitopes se liant de façon stable à l'une au moins des 9 molécules HLA de types suivants : A1, A2, A3, A11, A29, B8, B35, B44, ou B52, lesdits épitopes étant les suivants :
- (180)VLEWRFDSRL(189) se liant de façon stable aux molécules HLA de type A2,
- (182)EWRFDSRL(189) se liant de façon stable aux molécules HLA de type B8,
- (184)RFDSRLAF(191) se liant de façon stable aux molécules HLA de type A1,
- (186)DSRLAFHHV(194) se liant de façon stable aux molécules HLA de type B35,
- (188)RLAFHHVA(195) se liant de façon stable aux molécules HLA de type B52,
- (188)RLAFHHVAR(196) se liant de façon stable aux molécules HLA de type A3 ou A11,
- (190)AFHHVAREL(198) se liant de façon stable aux molécules HLA de type 852,
- (195)ARELHPEY(202) se liant de façon stable aux molécules HLA de type A1,
- (196)RELHPEYF(203) se liant de façon stable aux molécules HLA de type A29 ou B44.

2. Séquences nucléotidiques codant pour un fragment polyépitopique selon la revendications 1, corrrespondant aux séquences suivantes:
- la séquence SEQ ID NO : 3, codant pour le fragment polyépitopique SEQ ID NO : 4,
- la séquence SEQ ID NO : 5, codant pour le fragment polyépitopique SEQ ID NO : 6,
- la séquence SEQ ID NO : 7, codant pour le fragment polyépitopique SEQ ID NO : 8.

3. Lipopeptide **caractérisé en ce qu'**il comprend:
- une partie peptidique comprenant un ou plusieurs fragments protéiques polyépitopiques, tels que définis dans la revendication 1,
- et une ou plusieurs parties lipophiles comprenant :
* une chaîne hydrocarbonée en C4 à C20, saturée ou insaturée, linéaire ou ramifiée,
* ou un groupe stéroïde, le cas échéant lié à la chaîne hydrocarbonée susmentionnée.

4. Composition pharmaceutique, ou vaccin, **caractérisés en ce qu'**ils comprennent :
* a)
- au moins un fragment polyépitopique de la protéine Nef défini dans la revendication 1,
- et/ou des lipopeptides selon la revendication 1, et/ou des micelles, contenant au moins un fragment polyépitopique selon la revendication 1de la protéine Nef,
en association avec un véhicule physiologiquement acceptable,
ledit fragment protéique polyépitopique étant le cas échéant associés à un ou plusieurs autres épitopes reconnus par des cellules T auxiliaires, tels que le fragment peptidique délimité par les acides aminés situés aux positions 830 et 846 de la séquence peptidique de la toxine tétanique, l'hémagglutinine, ou l'épitope PADRE,
* ou b)
- au moins une séquence nucléotidique selon la revendication 2, codant pour un fragment polyépitopique selon la revendication 1 de la protéine Nef,
- et/ou au moins un vecteur approprié, contenant au moins une séquence nucléotidique susmentionnée, choisi notamment parmi les virus,
en association avec un véhicule physiologiquement acceptable.

5. Utilisation de fragments polyépitopiques de la protéine Nef comprenant les épitopes définis dans la revendication 1, ou de séquences nucléotidiques selon la revendication 2, ou de lipopeptides selon la revendication 4, pour la préparation d'un médicament ou vaccin destiné à la prévention ou au traitement du SIDA.

## Claims

1. Polyepitopic fragment of the Nef protein of HIV **characterized in that** it corresponds to:
* the fragment of 30 amino acids delimited by amino acids located at positions 68 and 97 of the peptide sequence of the Nef protein, and **characterized by** the peptide sequence SEQ ID NO: 4 as follows:
(68)FPVTPQVPLRPMTYKAAVDLSHFLKEKGGL(97)
said fragment containing 13 epitopes binding stably to at least one of the 9 HLA molecules of the following types: A2, A3, A11, B7, B8, B14, Cw8, B35, and B62, said epitopes being the following:
- (68)FPVTPQVPL(76) binding stably to HLA molecules of the type B7 or B35,
- (71)TPQVPLRPM(79) binding stably to HLA molecules of the type B35, or B7,
- (71)TPQVPLRPMTY(81) binding stably to HLA molecules of the type B7, or B35,
- (73)QVPLRPMTYK(82) binding stably to HLA molecules of the type A3 or A11,
- (74)VPLRPMTY(81) binding stably to HLA molecules of the type B35,
- (77)RPMTYKAAV(85) binding stably to HLA molecules of the type B7,
- (82)KAAVDLSHFL(91) binding stably to HLA molecules of the type B14 or Cw8,
- (83)AAVDLSHF(90) binding stably to HLA molecules of the type B62,
- (83)AAVDLSHFL(91) binding stably to HLA molecules of the type A2,
- (84)AVDLSHFL(91) binding stably to HLA molecules of the type A2,
- (84)AVDLSHFLK(92) binding stably to HLA molecules of the type A11,
- (86)DLSHFLKEK(94) binding stably to HLA molecules of the type A3,
- (90)FLKEKGGL(97) binding stably to HLA molecules of the type A2 or B8.
* the fragment of 30 amino acids delimited by the amino acids located at positions 116 and 145 of the peptide sequence of the Nef protein, and **characterized by** the peptide sequence SEQ ID NO: 6 as follows:
(116)HTQGYFPDWQNYTPGPGVRYPLTFGWCYKL(145)
said fragment containing 15 epitopes binding stably to at least one of the 14 HLA molecules of the following types: A1, A2, A3, A24, A32, B7, B18, B27, B35, B37, B49, B57, B58, or B62, said epitopes being the following:
- (116)HTQGYFPDW(124) binding stably to HLA molecules of the type B57 or B58,
- (117)TQGYFPDWQNY(127) binding stably to HLA molecules of the type B62,
- (120)YFPDWQNY(127) binding stably to HLA molecules of the type B37, B57 or B58,
- (121)FPDWQNYT(128) binding stably to HLA molecules of the type A1,
- (126)NYTPGPGVRY(135) binding stably to HLA molecules of the type A24,
- (128)TPGPGVRY(135) binding stably to HLA molecules of the type B35,
- (128)TPGPGVRYPL(137) binding stably to HLA molecules of the type B7 or B35,
- (130)GPGVRYPLTF(139) binding stably to HLA molecules of the type B35, or B7,
- (132)GVRYPLTFGW(141) binding stably to HLA molecules of the type B57,
- (133)VRYPLTFGW(141) binding stably to HLA molecules of the type A32 or B27,
- (134)RYPLTFGWCY(143) binding stably to HLA molecules of the type A24,
- (135)YPLTFGWCY(143) binding stably to HLA molecules of the type B7, B18, B35, or B49,
- (136)PLTFGWCYK(144) binding stably to HLA molecules of the type A3,
- (136)PLTFGWCYKL(145) binding stably to HLA molecules of the type A2,
- (137)LTFGWCYKL(145) binding stably to HLA molecules of the type A2.
* the fragment of 24 amino acids delimited by the amino acids located at positions 180 and 203 of the peptide sequence of the Nef protein, and **characterized by** the peptide sequence SEQ ID NO: 8 as follows:
(180)VLEWRFDSRLAFHHVARELHPEYF(203)
said fragment containing 9 epitopes binding stably to at least one of the 9 HLA molecules of the following types: A1, A2, A3, A11, A29, B8, B35, B44, or B52, said epitopes being the following:
- (180)VLEWRFDSRL(189) binding stably to HLA molecules of the type A2,
- (182)EWRFDSRL(189) binding stably to HLA molecules of the type B8,
- (184)RFDSRLAF(191) binding stably to HLA molecules of the type A1,
- (186)DSRLAFHHV(194) binding stably to HLA molecules of the type B35,
- (188)RLAFHHVA(195) binding stably to HLA molecules of the type B52,
- (188)RLAFHHVAR(196) binding stably to HLA molecules of the type A3 or A11,
- (190)AFHHVAREL(198) binding stably to HLA molecules of the type B52,
- (195)ARELHPEY(202) binding stably to HLA molecules of the type A1,
- (196)RELHPEYF(203) binding stably to HLA molecules of the type A29 or B44.

2. Nucleotide sequences coding for a polyepitopic fragment according to claim 1, corresponding to the following sequences:
- the sequence SEQ ID NO: 3, coding for the polyepitopic fragment SEQ ID NO: 4,
- the sequence SEQ ID NO: 5, coding for the polyepitopic fragment SEQ ID NO: 6,
- the sequence SEQ ID NO: 7, coding for the polyepitopic fragment SEQ ID NO: 8.

3. Lipopeptide **characterized in that** it comprises:
- a peptide portion comprising one or several polyepitopic protein fragments, as defined in claim 1,
- and one or several lipophile portions comprising:
* a C4 to C20 hydrocarbon chain, saturated or unsaturated, linear or branched,
* or a steroid group, as the case may be bound to the above-mentioned hydrocarbon chain.

4. Pharmaceutical composition, or vaccine, **characterized in that** they comprise:
* a)
- at least one polyepitopic fragment of the Nef protein defined in claim 1,
- and/or lipopeptides according to claim 1, and/or micelles, containing at least one polyepitopic fragment according to claim 1 of the Nef protein,
in association with a physiologically acceptable vehicle,
said polyepitopic protein fragment being as the case may be associated with one or several other epitopes recognized by T-helper cells, such as the peptide fragment delimited by the amino acids located at positions 830 and 846 of the peptide sequence of the tetanus toxin, hemagglutinin, or PADRE epitope,
* or b)
- at least one nucleotide sequence according to claim 2, coding for a polyepitopic fragment according to claim 1 of the Nef protein,
- and/or at least one suitable vector, containing at least one above-mentioned nucleotide sequence, selected particularly from viruses,
in association with a physiologically acceptable vehicle.

5. The use of polyepitopic fragments of the Nef protein comprising the epitopes as defined in claim 1, or of nucleotide sequences according to claim 2, or of lipopeptides according to claim 4, for the preparation of a drug or vaccine adapted for the prevention or treatment of AIDS.

## Patentansprüche

1. Polyepitop-Fragmente des Nef-Proteins des HIV, **dadurch gekennzeichnet, dass** sie:
* dem Fragment mit 30 Aminosäuren, das von den Aminosäuren begrenzt ist, die an den Positionen 68 und 97 der Peptidsequenz des Nef-Proteins liegen, und dass sie durch die folgende Peptidsequenz SEQ ID NO: 4:
(68) FPVTPQVPLRPMTYKAAVDLSHFLKEKGGL (97)
**gekennzeichnet** sind,
wobei das Fragment, das 13 Epitope enthält, sich auf stabile Weise an mindestens eines der 9 HLA-Moleküle der folgenden Typen bindet: A2, A3, A11, B7, B8, B14, Cw8, B35 und B62, wobei die Epitope Folgende sind:
- (68) FPVTPQVPL (76), das sich auf stabile Weise an die HLA-Moleküle vom Typ B7 oder B35 bindet,
- (71) TPQVPLRPM (79), das sich auf stabile Weise an die HLA-Moleküle vom Typ B35 oder B7 bindet,
- (71) TPQVPLRPMTY (81), das sich auf stabile Weise an die HLA-Moleküle vom Typ B7 oder B35 bindet,
- (73) QVPLRPMTYK (82), das sich auf stabile Weise an die HLA-Moleküle vom Typ A3 oder A11 bindet,
- (74) VPLRPMTY (81), das sich auf stabile Weise an die HLA-Moleküle vom Typ B35 bindet,
- (77) RPMTYKAAV (85), das sich auf stabile Weise an die HLA-Moleküle vom Typ B7 bindet,
- (82) KAAVDLSHFL (91), das sich auf stabile Weise an die HLA-Moleküle vom Typ B14 oder Cw8 bindet,
- (83) AAVDLSHF (90), das sich auf stabile Weise an die HLA-Moleküle vom Typ B62 bindet,
- (83) AAVDLSHFL (91), das sich auf stabile Weise an die HLA-Moleküle vom Typ A2 bindet,
- (84) AVDLSHFL (91), das sich auf stabile Weise an die HLA-Moleküle vom Typ A2 bindet,
- (84) AVDLSHFLK (92), das sich auf stabile Weise an die HLA-Moleküle vom Typ A11 bindet,
- (86) DLSHFLKEK (94), das sich auf stabile Weise an die HLA-Moleküle vom Typ A3 bindet,
- (90) FLKEKGGL (97), das sich auf stabile Weise an die HLA-Moleküle vom Typ A2 oder B8 bindet,
* dem Fragment mit 30 Aminosäuren, das von den Aminosäuren begrenzt ist, die an den Positionen 116 und 145 der Peptidsequenz des Nef-Proteins liegen, und durch die folgende Peptidsequenz SEQ ID NO: 6:
(116) HTQGYFPDWQNYTPGPGVRYPLTFGWCYKL (145)
**gekennzeichnet** sind,
wobei das Fragment, das 15 Epitope enthält, sich auf stabile Weise an mindestens eines der 14 HLA-Moleküle der folgenden Typen bindet: A1, A2, A3, A24, A32, B7, B18, B27, B35, B37, B49, B57, B58 oder B62, wobei die Epitope Folgende sind:
- (116) HTQGYFPDW (124), das sich auf stabile Weise an die HLA-Moleküle vom Typ B57 oder B58 bindet,
- (117) TQGYFPDWQNY (127), das sich auf stabile Weise an die HLA-Moleküle vom Typ B62 bindet,
- (120) YFPDWQNY (127), das sich auf stabile Weise an die HLA-Moleküle vom Typ B37, B57 oder B58 bindet,
- (121) FPDWQNYT (128), das sich auf stabile Weise an die HLA-Moleküle vom Typ A1 bindet,
- (126) NYTPGPGVRY (135), das sich auf stabile Weise an die HLA-Moleküle vom Typ A24 bindet,
- (128) TPGPGVRY (135), das sich auf stabile Weise an die HLA-Moleküle vom Typ B35 bindet,
- (128) TPGPGVRYPL (137), das sich auf stabile Weise an die HLA-Moleküle vom Typ B7 oder B35 bindet,
- (130) GPGVRYPLTF (139), das sich auf stabile Weise an die HLA-Moleküle vom Typ B35 oder B7 bindet,
- (132) GVRYPLTFGW (141), das sich auf stabile Weise an die HLA-Moleküle vom Typ B57 bindet,
- (133) VRYPLTFGW (141), das sich auf stabile Weise an die HLA-Moleküle vom Typ A32 oder B27 bindet,
- (134) RYPLTFGWCY (143), das sich auf stabile Weise an die HLA-Moleküle vom Typ A24 bindet,
- (135) YPLTFGWCY (143), das sich auf stabile Weise an die HLA-Moleküle vom Typ B7, B18, B35 oder B49 bindet,
- (136) PLTFGWCYK (144), das sich auf stabile Weise an die HLA-Moleküle vom Typ A3 bindet,
- (136) PLTFGWCYKL (145), das sich auf stabile Weise an die HLA-Moleküle vom Typ A2 bindet,
- (137) LTFGWCYKL (145), das sich auf stabile Weise an die HLA-Moleküle vom Typ A2 bindet.
* dem Fragment mit 24 Aminosäuren, das von den Aminosäuren begrenzt ist, die an den Positionen 180 und 203 der Peptidsequenz des Nef-Proteins liegen, und durch die folgende Peptidsequenz SEQ ID NO: 8:
(180) VLEWRFDSRLAFHHVARELHPEYF (203)
**gekennzeichnet** sind,
wobei das Fragment, das 9 Epitope enthält, sich auf stabile Weise an mindestens eines der 9 HLA-Moleküle der folgenden Typen bindet: A1, A2, A3, A11, A29, B8, B35, B44 oder B52, wobei die Epitope Folgende sind:
- (180) VLEWRFDSRL (189), das sich auf stabile Weise an die HLA-Moleküle vom Typ A2 bindet,
- (182) EWRFDSRL (189), das sich auf stabile Weise an die HLA-Moleküle vom Typ B8 bindet,
- (184) RFDSRLAF (191), das sich auf stabile Weise an die HLA-Moleküle vom Typ A1 bindet,
- (186) DSRLAFHHV (194), das sich auf stabile Weise an die HLA-Moleküle vom Typ B35 bindet,
- (188) RLAFHHVA (195), das sich auf stabile Weise an die HLA-Moleküle vom Typ B52 bindet,
- (188) RLAFHHVAR (196), das sich auf stabile Weise an die HLA-Moleküle vom Typ A3 oder A11 bindet,
- (190) AFHHVAREL (198), das sich auf stabile Weise an die HLA-Moleküle vom Typ B52 bindet,
- (195) ARELHPEY (202), das sich auf stabile Weise an die HLA-Moleküle vom Typ A1 bindet,
- (196) RELHPEYF (203), das sich auf stabile Weise an die HLA-Moleküle vom Typ A29 oder B44 bindet, entsprechen.

2. Nukleotidsequenzen, die für ein Polyepitopfragment nach Anspruch 1 kodieren, das den folgenden Sequenzen entspricht:
- der Sequenz SEQ ID NO: 3, die für das Polyepitopfragment SEQ ID NO: 4 kodiert,
- der Sequenz SEQ ID NO: 5, die für das Polyepitopfragment SEQ ID NO: 6 kodiert,
- der Sequenz SEQ ID NO: 7, die für das Polyepitopfragment SEQ ID NO: 8 kodiert.

3. Lipopeptid, **dadurch gekennzeichnet, dass** es:
- einen peptidischen Teil, der ein oder mehrere polyepitopische Proteinfragmente, wie in Anspruch 1 definiert, umfasst,
- und ein oder mehrere lipophile Teile umfasst, die:
* eine gesättigte oder ungesättigte, lineare oder verzweigte Kohlenwasserstoffkette mit 4 bis 20 C-Atomen
* oder eine Steroidgruppe, die gegebenenfalls an die oben genannte Kohlenwasserstoffkette gebunden ist, umfassen.

4. Pharmazeutische Zusammensetzung oder Vakzine, **dadurch gekennzeichnet, dass** sie:
* a)
- mindestens ein in Anspruch 1 definiertes Polyepitopfragment des Nef-Proteins,
- und/oder Lipopeptide nach Anspruch 1 und/oder Mizellen, die ein Polyepitopfragment nach Anspruch 1 des Nef-Proteins enthalten,
in Assoziation mit einem physiologisch verträglichen Träger,
wobei das polyepitopische Proteinfragment gegebenenfalls mit einem oder mehreren Epitopen assoziiert ist, die von den T-Helferzellen erkannt werden, wie etwa dem Peptidfragment, das von den Aminosäuren begrenzt wird, die an den Positionen 830 und 846 der Peptidsequenz des Tetanustoxins, des Hämagglutinins oder des Epitops PADRE liegen,
* oder b)
- mindestens eine Nukleotidsequenz nach Anspruch 2, die für ein Polyepitopfragment nach Anspruch 1 des Nef-Proteins kodiert,
- und/oder mindestens einen geeigneten Vektor, der mindestens eine oben genannte Nukleotidsequenz, insbesondere ausgewählt aus den Viren, enthält,
in Assoziation mit einem physiologisch verträglichen Träger umfassen.

5. Verwendung von Polyepitopfragmenten des Nef-Proteins, die in Anspruch 1 definierte Epitope oder Nukleotidsequenzen nach Anspruch 2 oder Lipopeptide nach Anspruch 4 umfassen, zur Herstellung eines Medikaments oder einer Vakzine, die zur Vorbeugung oder zur Behandlung von HIV bestimmt sind.
